# DEMANDE DE BREVET EUROPEEN

(11) **EP 4 141 984 A1**
(43) Date de publication de la demande: **01.03.2023**
(21) Numéro de dépôt: 22203057.9
(22) Date de dépôt: 09.06.2021
(51) Int. Cl.: H01M 4/04, H01M 4/139, H01M 4/1393, H01M 4/62, H01M 10/052

(54) **COMPOSITION POUR DES ELECTRODES DE BATTERIE**

(30) Priorité: 12.06.2020 FR 2006147
(62) Demande divisionnaire de: 21736634.3
(71) Demandeur: ARKEMA FRANCE, 92700 Colombes (FR)
(72) Inventeur: KORZHENDO, Alexander, 64170 LACQ (FR)

(57) **Abrégé**

La présente invention concerne une composition pour des électrodes de batteries dans laquelle au moins un solvant est une composition comprenant entre 80 et 95 % massique de N-méthylpyrrolidone (NMP).

## Description

La présente invention concerne une composition pour des électrodes de batteries dans laquelle au moins un solvant est une composition comprenant entre 80 et 95 % massique de N-méthylpyrrolidone (NMP).

Les compositions pour fabriquer les électrodes (cathode et anode) de batterie sont bien connues. Elles sont composées de solvant, de matière active, d'un conducteur électronique (par exemple de nature carbonée (noir de carbone, graphèmes, nanotubes de carbone, graphite, fibres de carbone, seuls ou en combinaison), d'un liant polymère, voire d'autres additifs tels qu'un ou plusieurs dispersant, polymères ou non.

Parmi les solvants utilisés on trouve la NMP pour ses propriétés adaptées (la capacité à dissoudre le liant polymère, et la température d'auto-inflammation très élevé (270°C) permettant l'application de la composition et le séchage en présence de l'air.

Ce solvant est généralement recyclé par distillation ou double distillation afin d'obtenir un maximum de degré de pureté en NMP. Cette opération vise à limiter la consommation de NMP dans le processus global de fabrication mais a cependant un cout sur le plan industriel. Jusqu'à ce jour, les procédés de fabrication d'électrode connus nécessitent un niveau de pureté d'au moins 99% massique en NMP. Lorsque l'on ne se souhaite pas recycler pour des raisons de coûts, le solvant est généralement brulé, ce qui est une aberration. Pour obtenir lors de l'opération de recyclage une pureté d'au moins 99% massique voir plus, on utilise par exemple des techniques de distillation décrites dans Ep2479167.

Dans le procédé de fabrication des électrodes de batterie on dépose une composition sur un collecteur de courant. Cette composition est constituée de solvant, de conducteur électronique, de charge active et de polymères pour les principaux. Ensuite, le solvant est évaporé pour obtenir l'électrode. La récupération de ce solvant ne permet pas d'obtenir une pureté telle que possible dans les procédés de distillation connus par exemple de EP2479167. Ce solvant récupéré lors du processus de fabrication d'électrode peut contenir jusqu'à près de 20 % de composés autre que du solvant pur.

Jusqu'ici les procédés industriels de fabrication d'électrodes nécessitent une purification du solvant ou l'utilisation de solvant « neuf ».

La demanderesse a constaté que dans le cas de la N-méthyle pyrrolidone, la réutilisation de ce solvant issu du procédé d'évaporation sous air lors de l'étape de fabrication des électrodes était non seulement possible sans purification, mais qu'elle présentait de nombreux avantages :
- Les dispersions sont meilleures.
- Les formulations sont plus stables dans le temps.
- Les électrodes fabriquées sont de meilleures qualité, plus précisément, présentent une meilleure répartition des composants carbonés dans les électrodes. Ainsi, la conductivité électrique est améliorée.

Cette NMP impure présente des teneurs en NMP comprise entre 80 et 99% et de préférence entre 95 et 99 %, des teneurs en humidité inférieures ou égales à 0.1%, et des composés issus des processus précédent d'utilisation de la NMP, typiquement des composés présentant des entités gamma-butyrolactame (2-pyrrolidone) ou gamma-butyrolactone.

La présence d'entités gamma-butyrolactame ou gamma-butyrolactone au sein des impuretés pourrait constituer des dispersants ou pourraient se greffer sur le conducteur électronique, facilitant ainsi sa répartition dans l'électrode. La demanderesse n'a pas d'explication et considère cet effet comme inattendu.

Ainsi, en conservant certains composés issus des processus précédents de fabrication des électrodes, non seulement on peut utiliser une telle NMP pour la fabrication de composition pour électrodes de batterie, mais qu'en plus les qualités des compositions obtenues sont supérieures.

La formulation d'électrode et plus particulièrement de cathode à base de tel solvant NMP, appliquée sur le collecteur de courant électrique subit un séchage sous air à la température au-dessous de l'ébullition de la NMP, typiquement en dessous de 220°C sous pression partielle. Dans les conditions de procédé identique de fabrication d'électrodes, l'utilisation de composition NMP de l'invention permet de diminuer la résistance électrique des électrodes, ce qui est un paramètre critique des applications batteries. De plus, l'utilisation de la composition de l'invention permet de simplifier la formulation des électrodes en réduisant par exemple la quantité de polymère dispersant et/ou liant.

### Résumé de l'invention :

L'invention concerne une composition comprenant :
- Au moins un solvant dont la composition comprend de la N-méthylpyrrolidone dans des teneurs massiques comprises entre 80 et 98,99 % bornes incluses, de l'eau dans des teneurs massiques inférieures à 1 % et au moins un composé possédant au moins un cycle gamma-butyrolactame ou gamma-butyrolactone, la NMP exclue, chacun de ces composés étant présent dans des proportions massiques allant de 0,01 % à 19 % bornes incluses seuls ou en combinaison, la totalité de ces composés n'excédant pas 19%, le dit solvant étant présent dans des proportions massiques supérieures ou égale à 50%.
- Au moins un conducteur électronique sous forme solide dans des proportions inférieures ou égales à 50 %, 0 exclu.

### Description détaillée :

Les compositions de l'invention comprennent moins de 50 % en masse de conducteur électronique sous forme solide, de préférence moins de 35 %, préférentiellement moins de 25 %, de façon encore préférée moins de 10 %, et de façon encore préférée moins de 5 % en masse.

Par conducteur électronique sous forme solide, on entend les noirs de carbone, les graphènes, les nanotubes de carbone mono ou multi paroi, les graphites, les fibres de carbone, seuls ou en combinaison. De préférence, il le conducteur électronique comprend des nanotubes de carbone.

Les compositions de l'invention comprennent également au moins un composé possédant au moins un cycle pyrrolidone (gamma-butyrolactame), la NMP exclue, et/ou gamma-butyrolactone, chacun de ces composés étant présent dans des proportions allant de 0,01 % à 19 % seuls ou en combinaison la totalité de ces composés n'excédant pas 19%.

Par composé possédant au moins un cycle pyrrolidone (gamma-butyrolactame) ou gamma-butyrolactone , il peut s'agir de tout composé d'un poids moléculaire inférieur à 15000 g/mole, de préférence inférieur à 10000 g/mole et dont la présence d'un cycle gamma-butyrolactone ou gamma-butyrolactame est caractérisée (spectro masse , RMN, spectro Infra-rouge). Les structures gamma-butyrolactone et gamma-butyrolactames peuvent porter des substituant comprenant C,H, N,O.

Parmi ces composés, on trouve la gamma butyro lactone, les succinimides tel que le N-methylsuccinimide, le N-hydroxysuccinimide, la formylpyrrolidone, la 5-hydroxy-Nmethylpyrrolidone, la 2-pyrrolidone, ou encore la N-hydroxymethylpyrrolidone parmis les principales. Il peut aussi s'agir d'oligomères porteur de cycle gamma-butyrolactame et/ gamma-butyrolactone de forme plus ou moins complexes ou difficile à caractériser tant dans leur nature que dans leur concentration.

On considère que toutes les impuretés dérivées de la structure gamma-butyrolactame ou gamma-butyrolactone présentent dans le solvant issu de l'évaporation lors du procédé de fabrication de l'électrode peuvent être présentes dans les compositions de l'invention. On peut également trouver des composés plus légers dont la structure ne comprend pas de structure gamma-butyrolactame ou gamma-butyrolactone.

De préférence, ces composés sont présents dans des teneurs comprises entre 0,01 % à 19 % massique, de façon encore préférée entre 0,1 et 10% et de façon encore préféré entre 0,5 % et 5 % massique, bornes incluses.

Cette composition de solvant est obtenue par récupération de vapeurs de NMP en présence d'air pour des températures supérieures à 120°.

Cette composition de solvant peut également être obtenue en rajoutant volontairement des dérivés de la gamma-butyrolactame et/ ou de la gamma-butyrolactone à de la NMP dont la pureté est supérieure à 99%, bien que ce ne soit pas l'objectif premier de l'invention qui utilise de la NMP sans besoin de purification présentant donc ces composés gamma-butyrolactones et/ou gamma-butyrolactames.

Les compositions de l'invention peuvent en outre comprendre une charge active. Par charge active on entend les oxydes des métaux de transition lithiés tels que LiMO₂, du type LiMPO₄, du type Li₂MPO₃F, du type Li₂MSiO₄ où M est Co, Ni, Mn, Fe ou une combinaison de ces derniers, du type LiMn₂O₄ ou du type S₈, les graphites artificiels ou naturels ou le silicium ou silicium modifiés carbure, nitrures, oxydes.

Les compositions de l'invention peuvent également comprendre un ou plusieurs polymères, choisi parmi les polymères de de poly(fluorure de vinylidène) (PVDF), la poly(vinyl pyrrolidone), le poly(phényl acétylène), le poly(meta-phénylène vinylidène), le polypyrrole, le poly(para-phénylène benzobisoxazole, le poly(alcool vinylique), la carboxy methyle cellulose et leurs mélanges + polyacrylonitrile est ces copolymères. De préférence, il s'agit de poly(fluorure de vinylidène) (PVDF) et de poly(N-vinyl pyrrolidone).

Les compositions de l'invention peuvent également comprendre un ou plusieurs générateurs de radicaux libres, tels que les peroxydes, les couples rédox, les composés azoiques ou encore les alcoxyamines, seuls ou en combinaison dans des teneurs inférieures à 5% et de préférence inférieure à 1% massique par rapport à la NMP.

L'invention concerne également un procédé d'obtention d'une électrode comprenant les étapes suivantes :
- Dépôt de la composition de l'invention comprenant au moins une charge active, au moins polymère sur un collecteur de courant électrique,
- Evaporation du solvant,
- Eventuellement calandrage.

L'invention concerne également un procédé d'obtention d'une électrode dans lequel le solvant récupéré par évaporation comprend le solvant de l'invention.

Le collecteur de courant électrique métallique est choisi parmi les métaux suivants : Al, Cu, Ni de façon non limitative, et présente une épaisseur comprise entre 8 et 35 µm. Le collecteur métallique peut aussi être revêtu d'un de primaire déposé sur le conducteur électronique avec une épaisseur comprise entre 0,5 et 5 µm.

Le calandrage consiste à compresser l'électrode entre deux rouleaux contrarotatifs, dont l'écart entres les rouleaux est inférieur à l'épaisseur de l'électrode.

L'électrode peut être une cathode ou une anode. De préférence il s'agit d'une cathode.

Pour les cathodes, la matière active est choisie parmi les oxydes des métaux de transition lithiés tels que LiMO₂, du type LiMPO₄, du type Li₂MPO₃F, du type Li₂MSiO₄ où M est Co, Ni, Mn, Fe ou une combinaison de ces derniers, du type LiMn₂O₄ ou du type S₈.

Pour les anodes, la matière active est choisie parmi les graphites artificiels ou naturels ou le silicium ou silicium modifiés carbure, nitrures, oxydes.

L'invention concerne également une batterie utilisant une cathode et/ou une anode obtenue selon de procédé de l'invention.

L'invention concerne également l'utilisation des compositions de l'invention dans les domaines tels que les encres et les peintures, ou dans l'extraction de pétrole.

La formulation des électrode (cathode ou anode) peut-être la suivante de façon non limitative:
- Un extra sec compris entre 20 et 90% massique, le reste est le solvant (composition comprenant de la N-méthylpyrrolidone dans des teneurs massiques comprises entre 80 et 98,99 % bornes incluses, de l'eau dans des teneurs massiques inférieures à 1 % et au moins un composé possédant au moins un cycle gamma-butyrolactame ou gamma-butyrolactone, la NMP exclue, chacun de ces composés étant présent dans des proportions massiques allant de 0,01 % à 19 % bornes incluses seuls ou en combinaison, la totalité de ces composés n'excédant pas 19% )
- Un ou plusieurs conducteurs électroniques dans des proportions comprises entre 0,1 et 5% de la formulation totale la formulation totale pour fabriquer l'électrode.
- Des additifs polymériques ou non (liant, dispersant) dans des proportions comprises entre 0,3 -5% de la formulation totale pour fabriquer l'électrode.

### Exemple 1 :

Préparation de la dispersion de Nanotubes de Carbone Graphistrength^{®} C100 HP dans le solvant NMP de qualité électronique (reference).

Les NTC Graphistrenth^{®} C100 HP est le grade commercial de ARKEMA contenant < 20 ppm des impuretés métalliques. Ce grade des NTC purifié est recommandé pour l'utilisation dans les Cathodes des Batteries Li Ion. Ces nanotubes de carbone sont multi parois (entre 10 et 15 parois) et leur surface spécifique est comprise entre 180 et 240 cm²/g.

### Préparation de la dispersion :

100 g de Graphistrength^{®} C100 HP sous forme de poudre sont pré-mélangés avec 400 g de NMP (qualité électronique, pureté > 99,8 % massique), à l'aide de défloculeur équipé d'une pale 55 mm.

L'ajout de 25 g de PVP K 30 (BASF) ce fait progressivement durant une heure de mélange à 1000-1600 rpm.

On rajoute ensuite 70 g de NMP après 30 min, et encore 65 g de NMP les 30 minutes suivantes.

La pré-dispersion en quantité de 660 g, contenant 15% de NTC et 3,8 % de PVP est prête pour l'étape de broyage en broyeur billes horizontal (BBH) de marque Brandt, à volume de chambre de broyage 250 ml.

Le broyeur est chargé avec 180 ml de billes céramiques d'un diamètre de 0.4-0.7 mm et le contre fer (gap size) est à 0.1-0.15 mm.

Le circuit de broyage est amorcé avec 230 g de NMP seule pendant 5 minutes. L'ajout progressif de la pré-dispersion se fait en 15-20 minutes, en montant la vitesse du rotor à 3000 rpm et pompe à 35% de la capacité.

Les mesures des extrais sec et de l'absorbance ont été réalisés pour suivre l'évolution de la dispersion (tableau 1).

### [Tableau 1]

**Tableau 1 :**

| Temps en minutes | Extrait Sec % | % massique NTC | Absorbance à 50 ppm NTC |
|---|---|---|---|
| 0 | 8.55 | 6.84 | 0.931 |
| 10 | 8.54 | 6.83 | 1.498 |
| | | | |
| 40 | 8.7 | 6.96 | 2.617 |
| 70 | 8.67 | 6.94 | 2.895 |
| 120 | 8.78 | 7.02 | 3.378 |
| | | | |
| 200 | 9.37 | 7.50 | 3.442 |
| 260 | 8.22 | 6.58 | 3.434 |

| Ajout de NMP pour viser 4% de NTC | | | |
|---|---|---|---|
| 320 | 4.93 | 3.94 | 3.346 |

Pour chaque mesure de l'absorbance, la dispersion prélevée du broyeur a été dilué à 50 ppm de NTC. Elle est mesurée à l'aide d'un spectromètre DR/2000 (HACH) à 355 nm de longueur d'ondes.

### Exemple 2 NMP de récupération et la dispersion de NTC à base de ce solvant (invention).

Le Solvant NMP de l'exemple 1, de qualité « électronique » a été utilisé pour réaliser la formulation « cathode » typique pour la batterie Li. Pour 960 g de NMC 622 produit par UMICORE (LiNi_{0.6}Mn_{0.2}Co_{0.2}O₂ ), 20 g de PVDF Kynar^{®} HSV 1810 produit par ARKEMA a été ajouté. Le pré- mélange sec a été dispersé dans 1950 ml de NMP à l'aide du mélangeur à disque durant 30 minutes. Ensuite, 50 g de la dispersion NTC de l'exemple 1 a été ajouté. Le solvant a été surdosé par rapport aux formulations utilisés dans la production des cathodes car le but de cette exemple est de modéliser la récupération du solvant en présence des ingrédients typiques d'une cathode.

La dispersion a été placé dans l'évaporateur rotative à 5 l de la marque Lab Rotovap. L'évaporateur à été chauffé à 145°C et le récipient du condensat a été maintenu à 110°C . La vanne de mise en dépression du condenseur a été maintenu ouverte pour garder le contact du condensat de NMP avec l'air.

En 24h 900 g de solvant ayant le couleur jeune prononcé a été récupéré dans le récipient.

La NMP récupérée a été analysée par la méthode de Karl Ficher pour la présence d'eau, dont la valeur obtenue est de 650 ppm. La quantité de NMP pure de 94,6% a été obtenu par la spectroscopie de masse. Ainsi, 5% env de NMP récupéré peuvent être attribués au produits d'oxydation de NMP non volatils.

Cet échantillon de NMP de récupération a été utilisé pour réaliser la dispersion NTC selon la méthode décrite dans l'exemple 1, en respectant les mêmes conditions.

Les mesures des extras et de l'absorbance ont été réalisés pour suivre l'évolution de la dispersion (tableau 2).

### [Tableau 2]

**Tableau 2 :**

| Temps en minutes | Extrait Sec % | % massique NTC | Absorbance à 50 ppm NTC |
|---|---|---|---|
| 0 | 8.51 | 6.81 | 1.022 |
| 15 | 8.64 | 6.91 | 1.634 |
| 40 | 8.67 | 6.94 | 2.801 |
| 60 | 8.81 | 7,05 | 3.244 |
| 90 | 8.85 | 7.08 | 3.645 |
| | | | |
| 120 | 9.51 | 7.61 | 3.845 |
| | | | |

| Ajout de NMP pour viser 4% de NTC | | | |
|---|---|---|---|
| 320 | 5,06 | 3.05 | 3.742 |

Pour le contrôle de l'absorbance, la NMP de récupération a été utilisée pour la cellule de référence. Nous constatons, que l'évolution de la dispersion est plus rapide dans le cas de l'NMP de récupération. Après 90 min de broyage l'absorbance approche la saturation, ce que nous observons après 120 min de broyage dans l'exemple 1. Le valeur d'absorbance est supérieur à l'exemple 1, ce qui peut se traduire par une meilleure performance de la dispersion de l'invention.

### Exemple 3. Performance électrique de la dispersion NTC de l'exemple 1 et 2 dans la formulation cathode

Préparation de la formulation Cathode. La solution de PVDF Kynar^{®} HSV 1810 à 8 % dans NMP de grade « électronique » en quantité de 12.5 g a été mélangée avec la même quantité de la dispersion NTC de l'exemple 1 à l'aide du mélangeur à disque, à 400 rpm. Après 15 min de mélange, on ajoute progressivement 98.5 g de NMC 622, 10g de NMP supplémentaire sont ajouter pour maintenir une bonne fluidité de la dispersion. L'objectif pour la viscosité est situé entre 3500 et 5000 cPs. La mélange a été complété après 30 min à 1500 rpm.

Ensuite, la dispersion a été appliquée sur un support en polyéthylène téréphtalate (PET) à 100 µm de l'épaisseur à l'aide de « doctor blade » en visant une épaisseur de revêtement de 120 µm. Le revêtement a été séché dans une étuve ventilée durant 30 min à 130°C.

L'extrait sec de cette formulation « modèle » de Cathode est suivante : NMC : 98.5% ; PVDF 1% ; NTC 0.5%

La feuille de PET revêtue est découpée pour obtenir les échantillons 3x4 cm. Les extrémités de chaque échantillon sont recouvertes d'une encre conductrice chargée en argent. La résistivité électrique a été mesurée à l'aide d'électromètre de la marque Keithlley.

Le même protocole a été appliqué pour obtenir un modèle de cathode avec la dispersion NTC de l'exemple 2. Les résultats des mesures électriques sont résumés dans le Tableau 3

### [Tableau 3]

**Tableau 3**

| | pureté NMP dans la dispersion NTC | qualité dispersion mesuré par absorbance | Conductivité électrode (cathode) Ohm cm |
|---|---|---|---|
| exemple comparatif NMP | > 99 % | 3,346 | 62 |
| exemple composition invention, NMP de récupération | < 94, 6% | 3,742 | 39 |

Dans les conditions similaires, la dispersion NTC réalisée à base de NMP de récupération démontre une meilleure performance électrique dans la formulation type de cathode NMC622, la conductivité de l'électrode est plus basse ce qui est favorable au bon fonctionnement d'une batterie.

## Revendications

1. composition comprenant :
- Au moins un solvant dont la composition comprend de la N-méthylpyrrolidone dans des teneurs massiques comprises entre 80 et 98,99 % bornes incluses, de l'eau dans des teneurs massiques inférieures à 1 % et au moins un composé possédant au moins un cycle gamma-butyrolactame ou gamma-butyrolactone, la NMP exclue, chacun de ces composés étant présent dans des proportions massiques allant de 0,01 % à 19 % bornes incluses seuls ou en combinaison, la totalité de ces composés n'excédant pas 19%, le dit solvant étant présent dans des proportions massiques supérieures ou égale à 50 %, le dit solvant étant obtenu par récupération de vapeurs de N-méthylpyrrolidone en présence d'air pour des températures supérieures à 120°.
- Au moins un conducteur électronique sous forme solide dans des proportions inférieures ou égales à 50 %, 0 exclu.

2. Composition selon la revendication 1 comprenant en outre une charge active et au moins un polymère.

3. Composition de l'une des revendications 1 à 2 comprenant en outre un générateur de radicaux libres tels que les peroxydes, les couples rédox, les composés azoiques ou encore les alcoxyamines, seuls ou en combinaison dans des teneurs inférieures à 5% massique.

4. Procédé d'obtention d'une électrode comprenant les étapes suivantes :
- Dépôt de la composition selon l'une des revendications 2 ou 3 sur un collecteur de courant électrique.
- évaporation du solvant ;
- éventuellement un calandrage

5. Electrode obtenue selon le procédé de la revendication 4.

6. Batterie obtenue avec une ou 2 électrodes selon la revendication 5.
